(19) Europäisches Patentamt
European Patent Office
Office européen des brevets

(11) **EP 3 984 450 B1**

(12) **EUROPEAN PATENT SPECIFICATION**

(45) Date of publication and mention
of the grant of the patent:
**04.06.2025  Bulletin 2025/23**

(51) International Patent Classification (IPC):
*A61B 5/00* (2006.01)    *A61B 5/021* (2006.01)
*A61B 5/024* (2006.01)    *A61B 5/1172* (2016.01)
*A61B 5/01* (2006.01)

(21) Application number: **21212750.0**

(22) Date of filing: **29.09.2016**

(52) Cooperative Patent Classification (CPC):
**A61B 5/02125; A61B 5/02427; A61B 5/1172;**
**A61B 5/6826; A61B 5/6843;** A61B 5/01

(54) **BLOOD PRESSURE MEASURING APPARATUS, AND BLOOD PRESSURE MEASURING APPARATUS USING LIGHT SOURCE SELECTION PROCESS**

BLUTDRUCKMESSVORRICHTUNG UND BLUTDRUCKMESSVORRICHTUNG MIT LICHTQUELLENAUSWAHLVERFAHREN

APPAREIL DE MESURE DE PRESSION ARTÉRIELLE ET APPAREIL DE MESURE DE PRESSION ARTÉRIELLE UTILISANT UN PROCESSUS DE SÉLECTION DE SOURCE LUMINEUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **02.10.2015  KR 20150139389**

(43) Date of publication of application:
**20.04.2022  Bulletin 2022/16**

(62) Document number(s) of the earlier application(s) in accordance with Art. 76 EPC:
**16191387.6 / 3 153 094**

(73) Proprietor: **Samsung Electronics Co., Ltd.**
**Suwon-si, Gyeonggi-do 16677 (KR)**

(72) Inventors:
• **KWON, Yong Joo**
**Gyeonggi-do (KR)**
• **KANG, Jae Min**
**Seoul (KR)**
• **KIM, Sun Kwon**
**Gyeonggi-do (KR)**
• **KIM, Youn Ho**
**Gyeonggi-do (KR)**

• **PARK, Sang Yun**
**Gyeonggi-do (KR)**

(74) Representative: **Grünecker Patent- und**
**Rechtsanwälte**
**PartG mbB**
**Leopoldstraße 4**
**80802 München (DE)**

(56) References cited:
EP-A1- 2 730 222          CN-A- 101 828 908
US-A1- 2005 228 296     US-A1- 2005 261 593
US-A1- 2009 043 180     US-A1- 2009 156 913
US-A1- 2013 137 938     US-A1- 2015 057 511
US-A1- 2015 157 262

• JING LIU ET AL: "Effects of cuff inflation and deflation on pulse transit time measured from ECG and multi-wavelength PPG", 2015 37TH ANNUAL INTERNATIONAL CONFERENCE OF THE IEEE ENGINEERING IN MEDICINE AND BIOLOGY SOCIETY (EMBC). PROCEEDINGS IEEE PISCATAWAY, NJ, USA, 29 August 2015 (2015-08-29), pages 5973 - 5976, XP002767819, ISBN: 978-1-4244-9270-1

## Description

## BACKGROUND

### 1. Field

Apparatuses and methods consistent with exemplary embodiments relate to a blood pressure measuring technology using light sources.

### 2. Description of the Related Art

A cuff-less blood pressure measuring apparatus measures blood pressure without pressurization. As general blood pressure estimating methods, a method of using a pulse wave velocity and a method of analyzing the shape of pulse waves are provided.

In the method of using a pulse wave velocity, the pulse wave velocity is measured by using a phase difference between an Electrocardiogram (ECG) signal and a Photo-Plethysmography (PPG) signal. In order to measure the ECG signal and the PPG signal, an electrode for the ECG signal and an optical measuring device for the PPG signal are required, such that it may be difficult to manufacture the measuring apparatus in a compact size. Further, both hands of a user may be required to contact the measuring apparatus to measure the ECG signal. JING LIU ET AL: "Effects of cuff inflation and deflation on pulse transit time measured from ECG and multi-wavelength PPG",2015 37TH ANNUAL INTERNATIONAL CONFERENCE OF THE IEEE ENGINEERING IN MEDICINE AND BIOLOGY SOCIETY (EMBC). PROCEEDINGS IEEE PISCATAWAY, NJ, USA, 29 August 2015 (2015-08-29), pages 5973-5976, ISBN: 978-1-4244-9270-1 studies the changes of PTTs and the relationship of the time difference between the peaks of different wavelength PPG and different PTTs. US 2009/0043180 A1 describes a pulse oximeter having a fingerprint reader.

## SUMMARY

One or more exemplary embodiments provide blood pressure measuring technology using a single light source, a plurality of light sources, and a light source selection process.

According to an aspect of an exemplary embodiment, there is provided a blood pressure measuring apparatus including: a light emitter configured to emit one or more lights having different penetration characteristics toward a user; light receiver configured to receive the lights that have penetrated through the user, and acquire photo-plethysmography (PPG) signals from the received lights; and a blood pressure measurer configured to measure a phase difference between the acquired PPG signals, and measure a blood pressure based on the measured phase difference.

The light emitter may include: a first light source configured to emit a first light in a range of an infrared wavelength to a red wavelength; and a second light source configured to emit a second light in a range of a blue wavelength to an ultraviolet wavelength.

The light emitter may further include a third light source configured to emit a third light in a range of a green wavelength.

The light receiver may acquire a first PPG signal from the first light, a second PPG signal from the second light, and a third PPG signal from the third light. The blood pressure measurer may measure a first phase difference between the first PPG signal and the second PPG signal, a second phase difference between the first PPG signal and the third PPG signal, and a third phase difference between the second PPG signal and the third PPG signal. The blood pressure measuring apparatus may calculate an average value of a first pulse wave velocity of the first PPG signal, a second pulse wave velocity of the second PPG signal, and a second pulse velocity of the third PPG signal based on the first phase difference, the second phase difference, and the third phase difference, and estimate a blood pressure based on the calculated average value.

The light emitter may include a single light source that emits a white light of a multi-wavelength band.

The light receiver may receive the white light penetrating though the user and filter the received white light by using a Red, Green and Blue (RGB) filter, and may acquire the PPG signal from the light filtered for each wavelength.

The light emitter may include, as the first light source, a light source that emits light having a first diffusion angle in a body of the user, and may include, as the second light source, a light source that emits a light having a second diffusion angle in the body. The second diffusion angle may be greater than the first diffusion angle.

The blood pressure measurer may calculate the pulse wave velocity based on the phase difference, and may estimate the blood pressure by using a relationship between the calculated pulse wave velocity and the blood pressure.

The blood pressure measuring apparatus may further include a pressure sensor configured to measure a tactile pressure input from the body, wherein the blood pressure measurer may further determine whether the measured tactile pressure is within a predetermined range of a tactile pressure.

The blood pressure measurer may determine a reference pressure within the predetermined range of the tactile pressure, and may calculate a correction coefficient for the measured tactile pressure based on the determined reference pressure.

The blood pressure measuring apparatus may further include a temperature sensor configured to measure a temperature of the body, wherein the blood pressure measurer may correct an error of the measured blood pressure based on the measured temperature and a relationship between the body temperature and the blood pressure.

According to the invention, there is provided a blood pressure measuring apparatus using a light source selection process, the apparatus including: a light source array comprising a plurality of light sources; a processor configured to selectively turn on one or more light sources from among the plurality of light sources to emit one or more lights toward a user; a light receiver configured to receive the lights that have penetrated through the user, and acquire photo-plethysmography (PPG) signals from the received lights; and a blood pressure measurer configured to measure a phase difference between the acquired PPG signals, and measure a blood pressure based on the measured phase difference.

The processor may select a first light source and a second light receiver from among the plurality of lights sources of the light source array. The second light source may be disposed closer to the light receiver than the first light source.

The processor may correct the phase difference based on a time delay between the PPG signals.

The blood pressure measuring apparatus includes a fingerprint recognition sensor configured to recognize fingerprints, wherein the processor may selectively turn on the one or more light sources based on at least one of a contact shape, a contact area, and a fingerprint pattern identified by the recognized fingerprints.

The processor may further provide the user with information about an appropriate contact position in which a finger of the user is to be placed to measure the blood pressure.

The processor may determine a predetermined position of a finger as a light emission position based on the fingerprint pattern, and may select the one or more light sources to emit the lights on the light emission position from among the plurality of light sources of the light source array.

The processor may determine the light emission position based on a location of a light source that maximizes the phase difference.

The processor may turn on a light source that is closer to the contact area than other light sources from among the plurality of light sources.

The processor may further include a storage configured to recognize individual users based on the recognized fingerprints, and store the measured blood pressure for the individual users.

The blood pressure measurer may calculate a pulse wave velocity based on the phase difference, and may estimate the blood pressure based on a relationship between the calculated pulse wave velocity and the blood pressure.

According to an aspect of another exemplary embodiment, there is provided a blood pressure measuring device including: a first light emitter configured to emit a first light of a first wavelength toward a subject; a second light emitter configured to emit a second light of a second wavelength that is shorter than the first wavelength toward the subject; a light detector configured to receive the first light passing through the subject after being emitted from the first light emitter and the second light passing through the subject after being emitted from the second light emitter; and a processor configured to detect a first photo-plethysmography (PPG) signal and a second PPG signal respectively from the received first light and the received second light, and determine a blood pressure of the subject based on a phase difference between the first PPG signal and the second PPG signal.

The blood pressure measuring device may further include a third light emitter configured to emit a third light of a third wavelength that is shorter than the first wavelength and longer than the second wavelength.

The second light emitter, the third light emitter, and the first light emitter may be respectively positioned in an order of increasing distance from the light detector.

The first light emitter, the second light emitter, the third light emitter correspond to a red light emitting diode (LED), a blue LED, and a green LED, respectively.

BRIEF DESCRIPTION OF THE DRAWINGS

The above and/or other aspects will be more apparent by describing certain exemplary embodiments, with reference to the accompanying drawings, in which:

FIG. 1 is a block diagram illustrating a blood pressure measuring apparatus according to an exemplary embodiment.

FIG. 2 is a diagram illustrating an example of a blood pressure measuring apparatus using a light source selection process according to another exemplary embodiment.

FIG. 3 is a diagram illustrating an example of emitting light on a finger.

FIG. 4 is a diagram illustrating an example of light, emitted from a light source, passing through a user's body part to be received by a light receiver.

FIG. 5 is a diagram illustrating an example of light, emitted from three light sources, passing through a user's body part to be received by a light receiver.

FIG. 6 is a diagram illustrating an example of penetration paths in a user's body of lights having different penetration characteristics.

FIG. 7 is a diagram illustrating an example of a filter layer of a PPG signal acquirer.

FIG. 8 is a diagram illustrating a graph of a phase difference between PPG signals.

FIG. 9 is a diagram illustrating a graph of a phase difference between three PPG signals.

FIG. 10 is a diagram illustrating an example of a blood pressure measuring apparatus further including a pressure sensor.

FIG. 11 is a diagram illustrating a graph showing a recommended range of a finger tactile pressure.

FIG. 12 is a diagram illustrating an example of a blood pressure measuring apparatus further including a transparent temperature sensor.

FIG. 13 is a diagram illustrating an example of a phase difference change depending on a temperature change.

FIG. 14 is a diagram illustrating an example of light, emitted from light sources that are located at different distances from a PPG signal acquirer, passing through a user's body to be received by a light receiver.

FIG. 15 is a diagram illustrating an example of selecting a light source located close to a contact area of a processor.

FIG. 16 is a diagram illustrating an example of measuring a phase difference between PPG signals.

FIG. 17 is a diagram illustrating graphs showing a phase difference at each wavelength.

DETAILED DESCRIPTION

Exemplary embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the exemplary embodiments. However, it is apparent that the exemplary embodiments can be practiced without those specifically defined matters. Also, well-known functions or constructions are not described in detail since they would obscure the description with unnecessary detail.

Expressions such as "at least one of," when preceding a list of elements, modify the entire list of elements and do not modify the individual elements of the list.

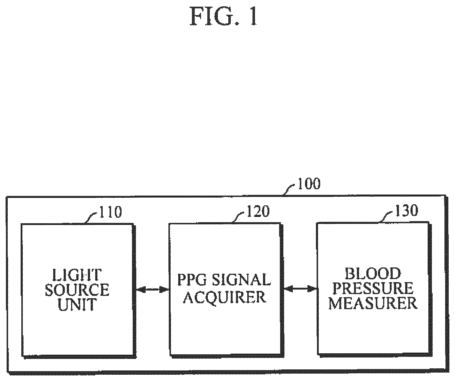

FIG. 1 is a block diagram illustrating a blood pressure measuring apparatus according to an exemplary embodiment. The blood pressure measuring apparatus 100 includes a light source unit 110, a photoplethysmography (PPG) signal acquirer 120, and a blood pressure measurer 130. The light source unit 110 and the PPG signal acquirer 120 may be implemented by a light emitter and a light receiver (or a light detector), respectively, The light source unit 110 and the PPG signal acquire 120 may be integrated into a single light emitter/receiver, or may be provided with two separate devices. Further, the blood pressure measuring apparatus 100 may include a pressure sensor and a temperature sensor.

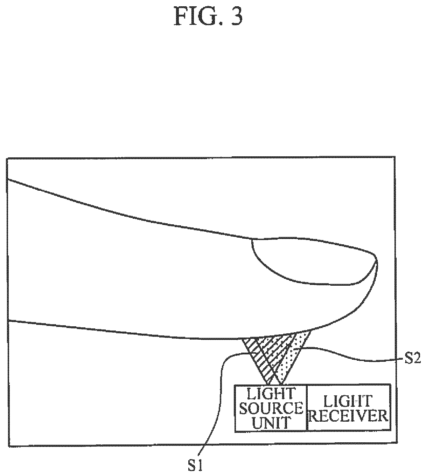

The light source unit 110 emits one or more lights having different characteristics of penetration into a user's body part, for example, a finger. Referring to FIG. 3, the light source unit 110 emits, onto a finger, a first light S1 and a second light S2 which have different penetration characteristics (e.g., penetration depth) from each other.

For example, light of different wavelengths may be transmitted to the body at different pulse wave velocities. In this case, the light source unit 110 includes a first light source, which emits the first light in a range of an infrared wavelength (e.g., 700 nm - 1 mm) to a red wavelength (e.g., 620-750 nm), and the second light source, which emits the second light in a range of a blue wavelength (e.g., 450-495 nm) to an ultraviolet wavelength (e.g., 10 nm - 400 nm). Further, the light source unit 110 may include a third light source that emits a third light in a range of a green wavelength (e.g., 495-570 nm). For example, the first light of a long wavelength, which is in a range of a red wavelength, may penetrate the skin and may reach, for example, blood vessels deep inside the skin, while the second light of a short wavelength may penetrate the skin to a shallow depth, to capillaries.

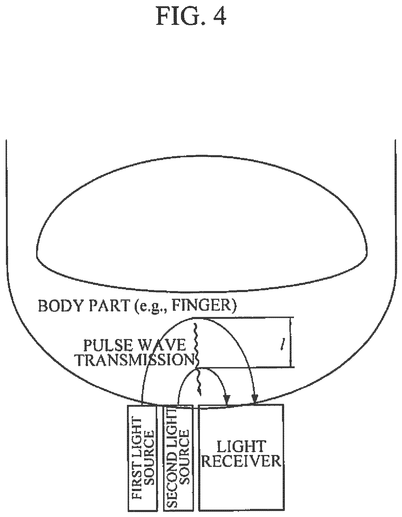

FIG. 4 is a diagram illustrating an example of light, emitted from a light source, passing through a user's body part to be received by a light receiver. Referring to FIG. 4, the first light source and the second light source each emit light on a user's body part, and the first light and the second light are transmitted through different paths in the body to the light receiver (e.g., PPG signal acquirer 120). Inside the body, the first light and the second light are spaced apart from each other at a distance ($l$). In this case, pulse wave velocities may vary depending on wavelength characteristics, and the first light and the second light, after passing through the body, may be received by the light receiver (e.g., PPG single acquirer 120) with a predetermined phase difference (PPT).

The first light is in a range of an infrared wavelength to a red wavelength, and has a long wavelength and a high pulse wave velocity. The second light is in a range of a blue wavelength to an ultraviolet wavelength, and has a lower pulse wave velocity than the first light. As the pulse wave velocity varies depending on wavelengths, light is received by the light receiver (e.g., PPG signal acquirer 120) with a phase difference.

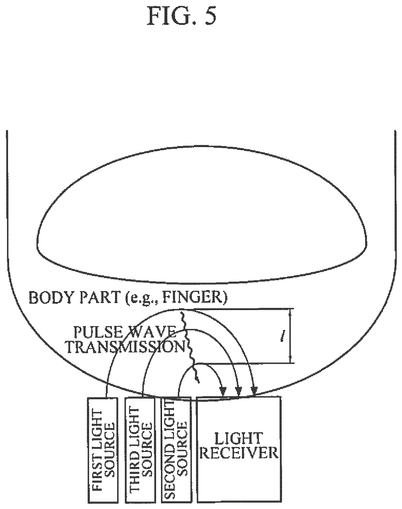

FIG. 5 is a diagram illustrating an example of light, emitted from three light sources, passing through a user's body part to be received by a light receiver. Referring to FIG. 5, a first light source, a second light source, and a third light source each emit light on a finger, and three emitted lights have different pulse wave velocities to penetrate into the skin to be received by the light receiver (e.g., PPG signal acquirer 120).

As an example of FIG. 5, the first light source of the light source unit 110 may emit a first light in a range of a red wavelength, the second light source of the light source unit 110 may emit a second light in a range of a blue wavelength, and the third light source of the light source unit 110 may emit a third light in a range of a green wavelength.

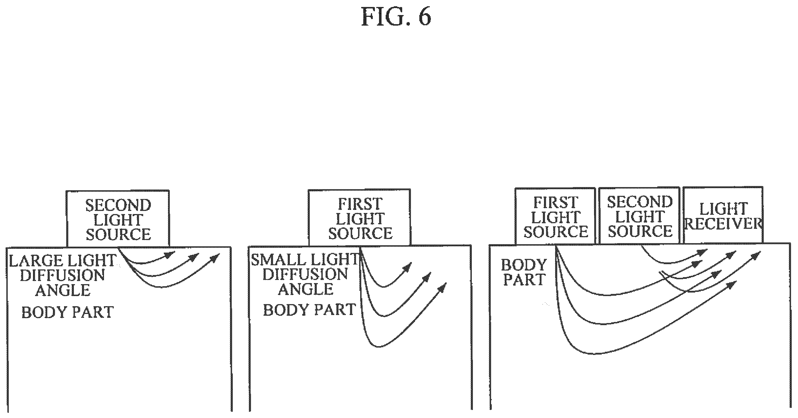

In another example, the light source unit 110 may include: as the first light source, a light source that emits light having a small diffusion angle in the body; and as the second light source, a light source that emits light having a larger diffusion angle in the body than the first light source. In this case, the light source, which emits light having a small diffusion angle, may be a laser diode (LD), and the light source, which emits light having a large diffusion angle, may be a light emitting diode (LED). FIG. 6 is a diagram illustrating an example of penetration paths in a user's body of lights having different penetration characteristics. The characteristics of light penetration into the body may include the length of a light wavelength, a diffusion angle of light, constituents according to the depth of body parts, light penetration speed, and the like.

The arrangement of the first light source, the second light source, and the third light source in relation to the light receiver may be determined based on the wavelength of each of the light sources. For example, the shorter the wavelength of the light source is, the closer the light source is positioned in relation to the light receiver. With reference to FIG. 5, the first light source, the second light source, and the third light source may correspond to a red LED, a blue LED, and a green LED, respectively, so that the blue LED, the green LED, and the red LED is disposed in the order of increasing their distance from the light receiver.

For example, the light source unit 110 may include a first light source and a second light source that have different diffusion angles in the body, in which a light source having a large diffusion angle may penetrate a shallow and wide part, while a light source having a small diffusion angle may penetrate a deep and narrow part.

Referring to FIG. 6, the light source unit 110 may include a laser diode (LD) and an LED as the first light source and the second light source, respectively. The diffusion angle of the LD is less than the diffusion angle of the LED. Each light emitted by the light source unit 110 penetrates a user's body through a different transmission path at a different penetration velocity, to be received by the light receiver (e.g., PPG signal acquirer 120). In another example, the light source unit 110 may emit white light including a multi-wavelength band. The white light is a single light and may include a multi-wavelength band, in which the PPG signal acquirer 120 may split the white light into different wavelengths of light.

The PPG signal acquirer 120 receives light that has passed through a user's body part, and may acquire a PPG signal from the received light. The PPG signal is a signal obtained by emitting light of a specific wavelength band on the body part and by detecting reflected or penetrated light, and a signal that indicates pulsation component generated according to a heartbeat rate.

For example, light emitted by the light source unit 110 is reflected from the body surface or passes through the body part, and is received by the light receiver of the PPG signal acquirer 120 with a phase difference. For example, a first light, which is emitted from the first light source and is in a range of a red wavelength, has a long transmission path in the body but a high pulse wave velocity, while a second light, which is emitted from the second light source and is in a range of a blue wavelength, has a short transmission path in the body but a low pulse wave velocity. The first light and the second light may be received by the PPG signal acquirer 120 with a phase difference.

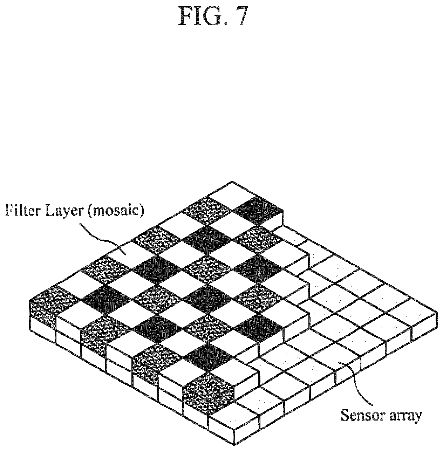

In another example, the PPG signal acquirer 120 may filter the single white light and pass only an allowed wavelength band of the light. FIG. 7 is a diagram illustrating an example of a filter layer of a PPG signal acquirer 120. Referring to FIG. 7, the PPG signal acquirer 120 may include an array of sensors, and a Red, Green and Blue (R-G-B) filter layer (mosaic). The RGB filter may filter light for red, green, and blue wavelengths. In this case, the PPG signal acquirer 120 may filter white light of a multi-wavelength band for each wavelength by using the RGB filter, and may acquire a PPG signal from each filtered light.

By using a single white light, and a single PPG signal acquirer 120, the blood pressure measuring apparatus 100 may be designed in a simple manner. The blood pressure measuring apparatus 100 of a simple design may be used in various applications, such as a smartphone, a tablet PC, a digital camera, a camera module, a wearable device, and the like.

The PPG signal acquirer 120 acquires a PPG signal from each received light. For example, the PPG signal acquirer 120 may receive a first PPG signal from the first light, a second PPG signal from the second light, a third PPG signal from the third light, and the like. The PPG signal acquirer 120 may receive a plurality of lights, and the number of which is not limited.

The PPG signal acquirer 120 may be a PPG sensor or an image sensor, and may also be a processor having a specific algorithm to acquire a PPG signal by processing a signal of the received light.

The blood pressure measurer 130 measures a phase difference (PPT) between feature points of acquired PPG signals, and may measure a blood pressure based on the measured phase difference. For example, the blood pressure measurer 130 may calculate a pulse wave velocity by extracting features points (e.g., peak points) by differentiating each PPG signal, and by measuring a phase difference (time difference) between the first PPG signal and the second PPG signal. Upon calculating the pulse wave velocity, the blood pressure measurer 130 may estimate a blood pressure based on a relationship between the pulse wave velocity and blood pressure. Blood pressure may be measured by such operations.

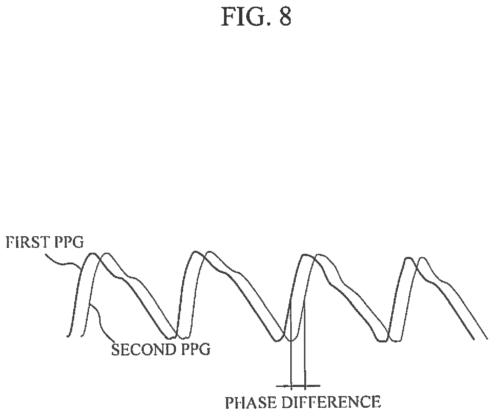

FIG. 8 is a diagram illustrating a graph of a phase difference between PPG signals. Referring to FIG. 8, a phase of the first PPG signal is acquired from light of a red wavelength and a phase of the second PPG signal is acquired from light of a blue wavelength that has a shorter wavelength than the red wavelength. The first PPG signal propagates faster than the second PPG signal. In other words, the phase velocity of the first PPG signal is greater than the phase velocity of the second PPG signal. In this case, the blood pressure measurer 130 may measure a phase difference between the first PPG signal and the second PPG signal.

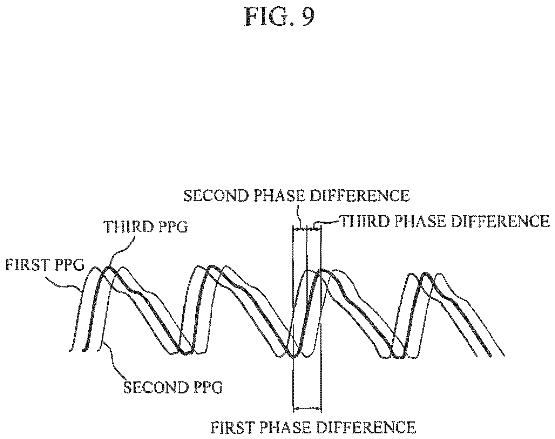

FIG. 9 is a diagram illustrating a graph of a phase difference between three PPG signals. In the exemplary embodiment, the blood pressure measurer 130 calculates a first pulse wave velocity based on a first phase difference between the first PPG signal and the second PPG signal; calculates a second pulse wave velocity based on a second phase difference between the first PPG signal and a third PPG signal; and calculates a third pulse wave velocity based on a third phase difference between the second PPG signal and the third PPG signal. The blood pressure measurer 130 may calculate an average of the three pulse wave velocities and may estimate a blood pressure based on the calculated average pulse wave velocity.

In addition, as the blood pressure measurer 130 may measure a phase difference among a plurality of PPG signals, and may calculate an average pulse wave velocity by using a plurality of phase differences, an error in measurement of phase difference may be reduced, and a blood pressure may be measured accurately.

Upon measuring a phase difference, the blood pressure measurer 130 may calculate a pulse wave velocity c by using the following Equation (1).

[Equation 1]

$$c = \frac{l}{\Delta T}$$

Here, length $l$ is a distance between the first PPG signal generator and the second PPG signal generator, and $\Delta T$ is a phase difference (time difference) between the first PPG signal and the second PPG signal. Since there is a direct relationship between a pulse wave velocity and a blood pressure, the blood pressure measurer 130 may estimate the blood pressure by calculating the pulse wave velocity.

In another example, the pulse wave velocity may be calculated by the following Equations (2) and (3).

[Equation 2]

$$E = E_0 e^{\alpha p}$$

[Equation 3]

$$c = \sqrt{\frac{Eh}{2\rho r}}$$

Here, E represents Young's modulus; $E_0$ represents Young's modulus at pressure 0; $\alpha$ represents a constant according to blood vessel characteristics; $p$ represents a blood pressure; $c$ represents a pulse wave velocity; $h$ represents a blood vessel thickness; $\rho$ represents a density; and $r$ represents a parameter based on a blood vessel radius. The pulse wave velocity c may be calculated by calculating or measuring these parameters.

Generally, when a blood pressure increases, the Young's modulus also increases, and the pulse wave velocity becomes faster. A PPG signal of the first light and a PPG signal of the second light have different penetration depths in the body, and when the PPG signals are measured at two specific points, a phase difference is caused between the two PPG signals.

The blood pressure measurer 130 may calculate the pulse wave velocity by using Equation (1) without applying the parameters of $p$, $h$, $\rho$, and $r$ in the calculation. Upon calculating the pulse wave velocity, the blood pressure measurer 130 may determine the blood pressure based on a relationship between the pulse wave velocity and the blood pressure.

Further, the blood pressure measuring apparatus 100 illustrated in FIG. 1 may further include at least one or more of a pressure sensor and a temperature sensor.

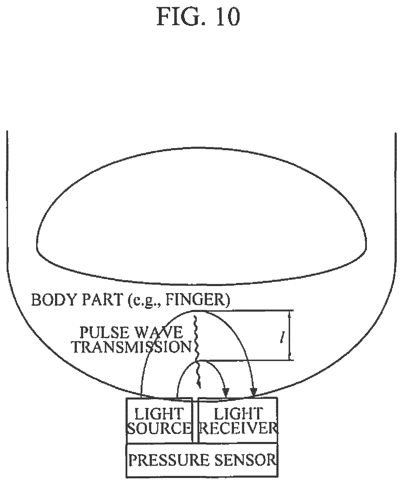

FIG. 10 is a diagram illustrating an example of a blood pressure measuring apparatus further including a pressure sensor. Referring to FIG. 10, once a user contacts a light source with his/her finger, the weight of the finger may be measured by a pressure sensor mounted at a lower portion of the light source and a light receiver. In this case, there may be a predetermined range of a tactile pressure that is appropriate for measurement of blood pressure, and the blood pressure measurer 130 may determine whether the measured pressure is within the predetermined range of the tactile pressure.

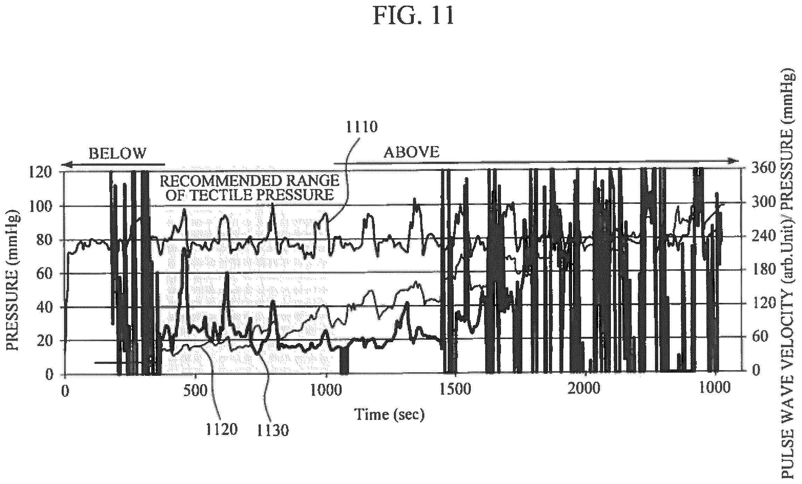

FIG. 11 is a diagram illustrating a graph showing a recommended range of a finger tactile pressure. The finger tactile pressure may be also referred to as a finger contact pressure. Referring to FIG. 11, when the pressure increases to reach a level that is below or above a predetermined range, the pulse wave velocity may not be calculated accurately. Generally, a pulse wave velocity graph and a blood pressure graph have the same shape and peak, and a blood pressure may be estimated by calculating a pulse wave velocity from the graphs. Referring to FIG. 11, however, as for levels of pressure that are not within the recommended range of the finger tactile pressure, the pulse wave velocities may show unstable peaks, rather than a predetermined shape of graph. That is, in the case where the measured pressure is not within the recommend range of the finger tactile pressure, the pulse wave velocity may not be calculated accurately, and a significant relationship between the pulse wave velocity and blood pressure may not be formed.

The blood pressure measurer 130 may determine whether a pressure, measured by the pressure sensor, is within the recommended range of the finger tactile pressure. When the measured pressure is beyond the recommended range, the blood pressure measuring apparatus 100 may generate a message that requests the user to adjust the finger tactile pressure. Further, even when the measured pressure is within the recommended range of the finger tactile pressure, a measurement error may be corrected according to an appropriate reference pressure. For example, the blood pressure measurer 130 may determine a reference pressure that is in the recommended range of the finger tactile pressure, and may calculate a correction factor for the measured pressure based on the determined reference pressure. For example, in the case where the measured pressure is greater than the reference pressure, the blood pressure measurer 130 may calculate a correction factor to correct the pulse wave velocity to be higher. By contrast, in the case where the measured pressure is less than the reference pressure, the blood pressure measurer 130 may calculate a correction factor to correct the pulse wave velocity to be lower. In this manner, the blood pressure measurer 130 may achieve an accurate blood pressure measurement regardless of a pressure exerted by a user. In the exemplary embodiment, a finger is used as a body part that may be in contact with the blood pressure measurer 130, but the present embodiment is not limited thereto. The user may use a different part of his/her body to contact the blood pressure measurer 130 and the contact pressure may be compared with a predetermined pressure range.

The blood pressure measuring apparatus 100 may further include an interface or an application, which informs a user of a finger tactile pressure measured by a pressure sensor, and provides an alarm function to a user so that an appropriate pressure may be input.

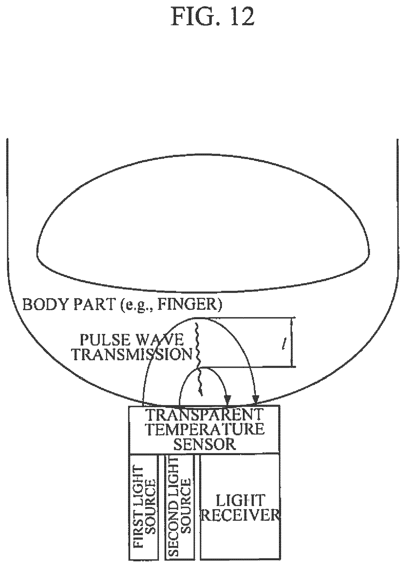

FIG. 12 is a diagram illustrating an example of a blood pressure measuring apparatus 100 which further includes a transparent temperature sensor that may measure the body temperature. Referring to FIG. 12, the transparent temperature sensor is disposed on the upper portion of a light source and a light receiver, to measure the body temperature without blocking light emitted from the light source.

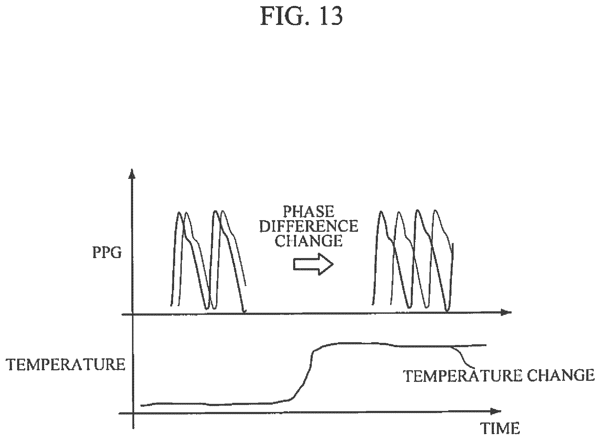

FIG. 13 is a diagram illustrating an example of a phase difference change depending on a temperature change. Referring to FIG. 13, the phase difference between the first PPG signal and the second PPG signal at a high temperature is greater than the phase difference at a low temperature. Once the temperature of a finger is measured by a temperature sensor 150, the blood pressure measurer 130 may correct an error of phase difference based on the measured temperature.

By using at least one of a pressure sensor and a temperature sensor, the blood pressure measuring apparatus 100 may check factors that affect a measurement result of a blood pressure, and may correct an error in the measurement result of the blood pressure.

The blood pressure measuring apparatus 100 may be included in a digital camera, an image sensor of a camera module, and the like, and may also be mounted in a smartphone, a tablet PC, a wearable device, a healthcare product, and the like.

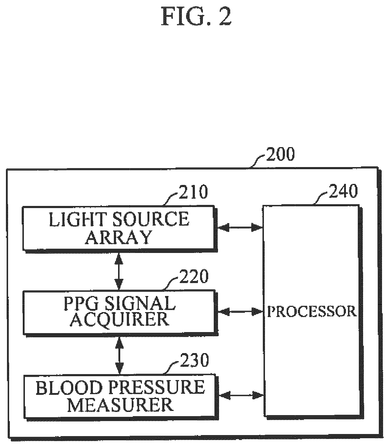

FIG. 2 is a diagram illustrating an example of a blood pressure measuring apparatus using a light source selection process according to another exemplary embodiment. The blood pressure measuring apparatus 200 using a light source selection process includes a light source array 210, a PPG signal acquirer 220, a blood pressure measurer 230, and a processor 240. The blood pressure measurer 230 may be integrated with the processor 240, or another processor separately provided from the processor 240. Further, the blood pressure measuring apparatus 200 using a light source selection process may further include a fingerprint recognition sensor, a pressure sensor, and a temperature sensor, in which elements that overlap with those illustrated in FIG. 1 will be briefly described hereinafter.

The light source array 210 may include a plurality of light sources, including a fist light source, a second light source, a third light source, and the like, which have different wavelengths. Further, the light source array 210 may include a laser diode and a light emitting diode (LED) which have different penetration characteristics in the body. In addition, the light source array 210 may include a plurality of single light sources, each emitting a single light such as white light.

The light source array 210 may arrange a plurality of light sources in a predetermined form or array. However, the arrangement form or array of the plurality of light sources in the light source array 210 is not limited, and there may be various array forms.

A processor 240 selects one or more light sources from among the plurality of light sources of the light source array 210, and may control the selected one or more light sources to be emitted on the body. Based on different penetration characteristics, the processor 240 may select one or more light sources by differing any one of a range of wavelengths, a range of diffusion angles, types of light sources, and locations of light sources.

For example, among the light sources of the light source array 210, the processor 240 may select the first light source located far from the PPG signal acquirer 220, or may select the second light source located closer to the PPG signal acquirer 220 than the first light source. The wavelength of the first light source may be greater than the wavelength of the second light source.

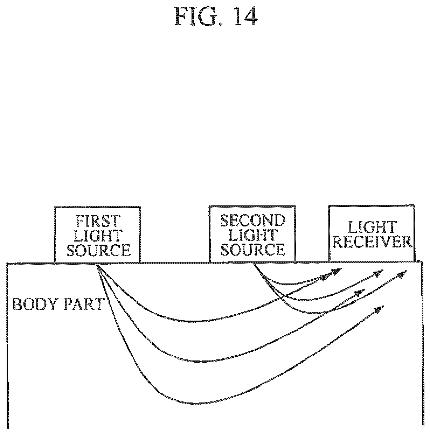

FIG. 14 is a diagram illustrating an example of light, emitted from light sources that are located at different distances from a PPG signal acquirer 220, passing through a user's body to be received by a light receiver. Referring to FIG. 14, the first light source, which is relatively farther from the PPG signal acquirer 220 (e.g., a light receiver), has a long transmission path in the body. Similarly, the second light source, which is relatively closer to the PPG signal acquirer 220, has a short transmission path. That is, depending on the location of light sources, there may be a phase difference between the first light source and the second light source that are received by the PPG signal acquirer 220 (e.g., the light receiver).

For example, the processor 240 may select the first light source, which is far from the PPG signal acquirer 220, and the second light source, which is close to the PPG signal acquirer 220. Referring to FIG 14, the distance between the first light source and the PPG signal acquirer 220 is longer than the distance between the second light source and the PPG signal acquirer 220, which may delay time for the first light to reach the light receiver. In this case, considering the time delay caused by different distances from the PPG signal acquirer 220, the processor 240 may correct a phase difference between the PPG signals.

Further, the blood pressure measuring apparatus 200 using a light source selection process may further include a fingerprint recognition sensor that recognizes fingerprints, in which the processor 240 may select a light source based on at least one of a contact shape, a contact area, and a fingerprint pattern, which are identified by the recognition of fingerprints.

For example, once the fingerprint recognition sensor recognizes a user's fingerprints, the processor 240 analyzes a contact shape, a contact area, and a fingerprint pattern, which are identified by the fingerprint recognition, and may identify a contact position of a finger.

In this case, the processor 240 may provide a user with information on an appropriate contact position of a finger, which is required to measure blood pressure. For example, the processor 240 may provide a user with the contact position of a finger, which is identified by fingerprint recognition, and a pre-stored appropriate contact position of a finger, which is required to measure blood pressure, through an interface. Further, the processor 240 may provide guide information to guide a user's finger to an appropriate position to measure blood pressure.

Among the light sources of the light source array 210, the processor 240 may select a light source that is located at an optimal location to emit light on the identified contact position of a finger.

For example, based on a finger pattern, the processor 240 may determine a position of a finger to be a light emission position, and may select a light source to emit light on the position from among the light sources of the light source array 210. For example, the processor 240 may determine a light emission position based on a location of a light source that maximizes a phase difference, in which the phase difference may be measured by experiments or by repetition, or may be calculated based on a location of a light source.

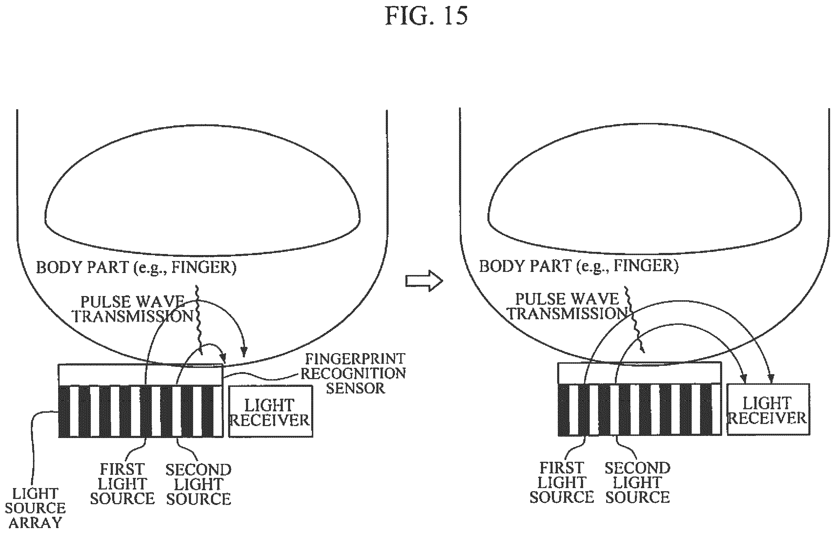

In another example, the processor 240 may select a light source that is close to a contact area. FIG. 15 is a diagram illustrating an example of selecting a light source located close to a contact area of a processor. Referring to FIG. 15, the light source array 210 may select two or more light sources having a wide contact area for a finger, and the processor 240 may select two or more light sources appropriately from among the light sources of the light source array 210.

In addition, the processor 240 may include a storage that recognizes individual users by recognition of fingerprints, and stores the measured blood pressure for each individual user.

The PPG signal acquirer 220 may receive light that has passed through the body part, and may acquire a Photo-plethysmography (PPG) signal from the received light.

The blood pressure measurer 230 may measure a phase difference between feature points of the acquired PPG signals, and may measure blood pressure based on the phase difference. For example, the blood pressure measurer 230 may calculate a pulse wave velocity by extracting features points (e.g., peak points) by differentiating each PPG signal, and by measuring a phase difference (time difference) between the first PPG signal and the second PPG signal. Upon calculating the pulse wave velocity, the blood pressure measurer 230 may estimate a blood pressure based on a relationship between the pulse wave velocity and the blood pressure. Blood pressure may be measured by such operations.

The blood pressure measuring apparatus 200 illustrated in FIG. 2 may identify states and conditions of blood pressure measurement by checking one or more of a fingerprint recognition sensor, a pressure sensor, and a temperature sensor, and may guarantee objectivity of blood pressure measurement.

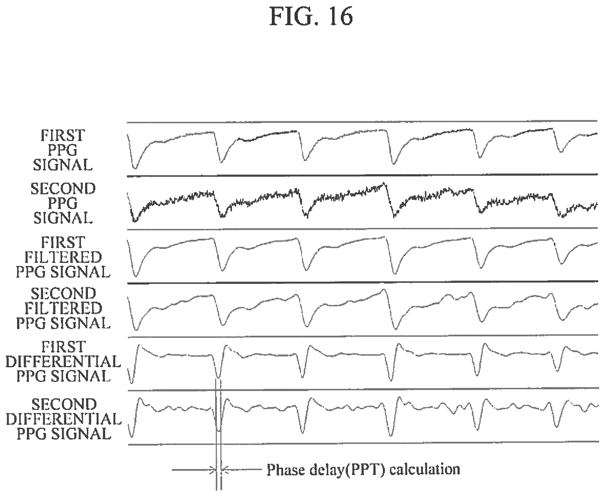

FIG. 16 is a diagram illustrating an example of measuring a phase difference between PPG signals. The blood pressure measurers 130 and 230 illustrated in FIGS. 1 and 2 may extract feature points from PPG signals, and may measure a phase difference between the feature points. For example, the blood pressure measurers 130 and 230 perform resampling of each PPG signal, for example, resampling of a frequency of 250 Hz to 2500 Hz, and pass the PPG signal through a low pass filter (LPF) to pass a frequency of lower than 10 Hz.

Referring to FIG. 16, the blood pressure measurers 130 and 230 filter graphs of the first PPG signal and the second PPG signal through the LPF, and may acquire the first and second filtered PPG signals. The first and second filtered PPG signals become graphs in a smoother shape than graphs before filtering. Further, the blood pressure measurers 130 and 230 extract feature points from graphs obtained by differentiating the first filtered PPG signal and the second filtered PPG signal. Upon extracting the feature points, the blood pressure measurers 130 and 230 may measure a phase difference between the first PPG signal and the second PPG signal.

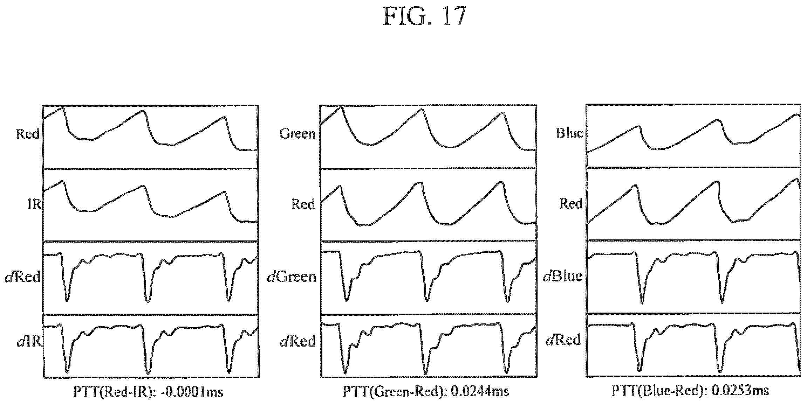

FIG. 17 is a diagram illustrating a graph showing a phase difference at each wavelength. Referring to FIG. 17, graphs show a phase difference between a red wavelength and an infrared wavelength, a phase difference between a red wavelength and a green wavelength, and a phase difference between a red wavelength and a blue wavelength. The blood pressure measurers 130 and 230 may extract feature points of each graph by differentiating each graph, and may measure a phase difference based on the feature points of each graph.

Referring to the left portion of FIG. 17, a phase difference between feature points of a differential graph (dRed) of a red wavelength and a differential graph (dIR) of an infrared wavelength is -0.0001 ms. In another example, referring to the middle portion of FIG. 17, a phase difference between feature points of a differential graph (dGreen) of a green wavelength and a differential graph (dRed) of a red wavelength is 0.0244 ms. In yet another example, referring to the right portion of FIG. 17, a phase difference between feature points of a differential graph (dBlue) of a blue wavelength and a differential graph (dRed) of a red wavelength is 0.0253 ms.

However, the above examples are merely illustrative, and red, blue and green wavelengths are selected randomly, and blood pressure may be measured by using other ranges of wavelengths.

According to the foregoing exemplary embodiment, a blood pressure measuring apparatus may have a simple configuration, and may minimize errors in the measurement of blood pressure in various circumstances.

While not restricted thereto, the operations or steps of the methods or algorithms according to the above exemplary embodiments may be embodied as computer-readable codes on a computer-readable recording medium. The computer-readable recording medium may be any recording apparatus capable of storing data that is read by a computer system. Examples of the computer-readable recording medium include read-only memories (ROMs), random-access memories (RAMs), CD-ROMs, magnetic tapes, floppy disks, and optical data storage devices. The computer-readable recording medium may be a carrier wave that transmits data via the Internet, for example. The computer-readable medium may be distributed among computer systems that are interconnected through a network so that the computer-readable code is stored and executed in a distributed fashion. Also, the operations or steps of the methods or algorithms according to the above exemplary embodiments may be written as a computer program transmitted over a computer-readable transmission medium, such as a carrier wave, and received and implemented in general-use or special-purpose digital computers that execute the programs. Moreover, it is understood that in exemplary embodiments, one or more units of the above-described apparatuses and devices can include or implemented by circuitry, a processor, a microprocessor, etc., and may execute a computer program stored in a computer-readable medium.

**The following are further embodiments.**

1. A blood pressure measuring apparatus comprising:

a light emitter configured to emit one or more lights having different penetration characteristics toward a user;
a light receiver configured to receive the lights that have penetrated through the user, and acquire photo-plethysmography (PPG) signals from the received lights; and
a blood pressure measurer configured to measure a phase difference between the acquired PPG signals, and measure a blood pressure based on the measured phase difference.

2. The apparatus of embodiment 1, wherein the light emitter comprises:

a first light source configured to emit a first light in a range of an infrared wavelength to a red wavelength; and
a second light source configured to emit a second light in a range of a blue wavelength to an ultraviolet wavelength.

3. The apparatus of embodiment 2, wherein the light emitter comprises a third light source configured to emit a third light in a range of a green wavelength, in particular, wherein:

the light receiver acquires a first PPG signal from the first light, a second PPG signal from the second light, and a third PPG signal from the third light;
the blood pressure measurer measures a first phase difference between the first PPG signal and the second PPG signal, a second phase difference between the first PPG signal and the third PPG signal, and a third phase difference between the second PPG signal and the third PPG signal,
wherein the blood pressure measuring apparatus calculates an average value of a first pulse wave velocity of the first PPG signal, a second pulse wave velocity of the second PPG signal, and a third pulse velocity of the third PPG signal based on the first phase difference, the second phase difference, and the third phase difference, and estimates a blood pressure based on the calculated average value.

4. The apparatus of embodiment 1, wherein the light emitter comprises a single light source that emits a white light of a multi-wavelength band.

5. The apparatus of embodiment 4, wherein the light receiver is further configured to receive the white light penetrating though the user, filter the received white light by using a Red, Green and Blue (RGB) filter, and acquire the PPG signal from the light filtered for each wavelength.

6. The apparatus of embodiment 2, wherein the light emitter comprises, as the first light source, a light source that emits a light having a first diffusion angle in a body of the user, and comprises, as the second light source, a light source that emits a light having a second diffusion angle in the body, the second diffusion angle being greater than the first diffusion angle.

7. The apparatus of embodiment 1, wherein the blood pressure measurer calculates a pulse wave velocity of the PPG signals based on a phase difference between the PPG signals, and estimates the blood pressure based on a relationship between the calculated pulse wave velocity and the blood pressure.

8. The apparatus of embodiment 1, further comprising a pressure sensor configured to measure a tactile pressure input from the body, wherein the blood pressure measurer further determines whether the measured tactile pressure is within a predetermined range of a tactile pressure, in particular, wherein the blood pressure measurer determines a reference pressure within the predetermined range of the tactile pressure, and calculates a correction coefficient for the measured tactile pressure based on the determined reference pressure.

9. The apparatus of embodiment 1, further comprising a temperature sensor configured to measure a temperature of the body, wherein the blood pressure measurer corrects an error of the measured blood pressure based on the measured temperature and a relationship between the body temperature and the blood pressure.

10. The apparatus of embodiment 1,

the light emitter comprising a light source array comprising a plurality of light sources; the apparatus further comprising a processor configured to selectively turn on one or more light sources from among the plurality of light sources to emit the one or more lights toward a user.

11. The apparatus of embodiment 10,

wherein the blood pressure measurer calculates a pulse wave velocity based on the phase difference, and estimates the blood pressure based on a relationship between the calculated pulse wave velocity and the blood pressure, and/or
wherein the processor is further configured to select a first light source and a second light source from among the plurality of lights sources of the light source array, and wherein the second light source is disposed closer to the light receiver than the first light source, in particular, wherein the processor is further configured to correct the phase difference based on a time delay between the PPG signals.

12. The apparatus of embodiment 10, further comprising a fingerprint recognition sensor configured to recognize fingerprints, wherein the processor is further configured to selectively turn on the one or more light sources based on at least one of a contact shape, a contact area, and a fingerprint pattern identified by the recognized fingerprints.

13. The apparatus of embodiment 12,

wherein the processor is further configured to provide the user with information about an appropriate contact position in which a finger of the user is to be placed to measure the blood pressure, and/or
wherein the processor is further configured to turn on a light source that is closer to the contact area than other light sources from among the plurality of light sources, and/or wherein the processor comprises a storage configured to recognize individual users based on the recognized fingerprints, and store the measured blood pressure for the individual users, and/or
wherein the processor is further configured to determine a predetermined position of a finger as a light emission position based on the fingerprint pattern and turn on the one or more light sources to emit the lights on the light emission position from among the plurality of light sources of the light source array, in particular, wherein the processor is further configured to determine the light emission position based on a location of a light source that maximizes the phase difference.

14. The apparatus of embodiment 1,

   wherein the one or more lights comprise a first light of a first wavelength and a second light of a second wavelength that is shorter than the first wavelength;
   wherein the PPG signals comprise a first photo-plethysmography (PPG) signal and a second PPG signal respectively from the received first light and the received second light; and
   wherein the phase difference is a phase difference between the first PPG signal and the second PPG signal.

15. The blood pressure measuring device of embodiment 14, wherein the one or more lights further comprise a third light of a third wavelength that is shorter than the first wavelength and longer than the second wavelength,

   in particular,
   wherein the first light is emitted by a first light emitter, the second light is emitted by a second light emitter, and the third light is emitted by a third light emitter, and

      wherein the second light emitter, the third light emitter, and the first light emitter are respectively positioned in an order of increasing distance from the light detector, and/or
      wherein the first light emitter, the second light emitter, the third light emitter correspond to a red light emitting diode (LED), a blue LED, and a green LED, respectively.

**Claims**

1. A blood pressure measuring apparatus (200) using a light source selection process, the apparatus comprising:

      a light source array (210) comprising a plurality of light sources;
      a light receiver configured to receive the lights that have penetrated through the user, and acquire photo-plethysmography (PPG) signals from the received lights; and
      a processor (240) configured to selectively turn on one or more light sources from among the plurality of light sources to emit one or more lights toward a user, and measure a phase difference between the acquired PPG signals, and measure a blood pressure based on the measured phase difference,
      a fingerprint recognition sensor configured to recognize fingerprints,

   wherein the processor is further configured to selectively turn on the one or more light sources based on at least one of a contact shape, a contact area, and a fingerprint pattern identified by the recognized fingerprints.

2. The apparatus of claim 1, wherein the processor is further configured to select a first light source and a second light source from among the plurality of lights sources of the light source array,
   wherein the second light source is disposed closer to the light receiver than the first light source.

3. The apparatus of claim 2, wherein the processor is further configured to correct the phase difference based on a time delay between the PPG signals.

4. The apparatus of claim 1, wherein the processor is further configured to provide the user with information about an appropriate contact position in which a finger of the user is to be placed to measure the blood pressure.

5. The apparatus of claim 1, wherein the processor is further configured to:

      determine a predetermined position of a finger as a light emission position based on the fingerprint pattern; and
      turn on the one or more light sources to emit the lights on the light emission position from among the plurality of light sources of the light source array.

6. The apparatus of claim 5, wherein the processor is further configured to determine the light emission position based on a location of a light source that maximizes the phase difference.

7. The apparatus of claim 1, wherein the processor is further configured to turn on a light source that is closer to the contact area than other light sources from among the plurality of light sources.

8. The apparatus of claim 1, wherein the processor comprises a storage configured to recognize individual users based on the recognized fingerprints, and store the measured blood pressure for the individual users.

9. The apparatus of claim 1, wherein the processor calculates a pulse wave velocity based on the phase difference, and estimates the blood pressure based on a relationship between the calculated pulse wave velocity and the blood pressure.

**Patentansprüche**

1. Blutdruck-Messvorrichtung (200), die ein Lichtquellen-Auswahlverfahren verwendet, wobei die Vorrichtung umfasst:

   eine Lichtquellengruppe (210), umfassend eine Vielzahl von Lichtquellen; einen Lichtempfänger, ausgebildet zum Empfangen der Lichtstrahlen, die durch den Benutzer durchgedrungen sind, und Erfassen von Photo-Plethysmography-(PPG-)Signalen aus den empfangenen Lichtstrahlen; und
   einen Prozessor (240), ausgebildet zum selektiven Einschalten von einer oder mehreren Lichtquellen aus der Vielzahl von Lichtquellen einschaltet, um ein oder mehrere Lichter in Richtung eines Benutzers auszustrahlen, und Messen einer Phasendifferenz zwischen den erfassten PPG-Signalen sowie Messen eines Blutdrucks basierend auf der gemessenen Phasendifferenz,
   einen Fingerabdruck-Erkennungssensor, ausgebildet zum Erkennen von Fingerabdrücken, wobei der Prozessor ferner zum selektiven Einschalten der einen oder mehreren Lichtquellen basierend auf wenigstens einem Element der Gruppe umfassend eine Kontaktform, eine Kontaktfläche und ein Fingerabdrucksmuster, identifiziert durch die erkannten Fingerabdrücke, ausgebildet ist.

2. Vorrichtung nach Anspruch 1, wobei der Prozessor ferner zum Auswählen einer ersten Lichtquelle und einer zweiten Lichtquelle aus der Vielzahl von Lichtquellen der Lichtquellengruppe ausgebildet ist, wobei die zweite Lichtquelle näher am Lichtempfänger angeordnet ist als die erste Lichtquelle.

3. Vorrichtung nach Anspruch 2, wobei der Prozessor ferner zum Korrigieren der Phasendifferenz basierend auf einer Zeitverzögerung zwischen den PPG-Signalen ausgebildet ist.

4. Vorrichtung nach Anspruch 1, wobei der Prozessor ferner zum Versorgen des Benutzers mit Informationen zu einer geeigneten Kontaktposition, in der ein Finger des Benutzers zum Messen des Blutdrucks anzuordnen ist, ausgebildet ist.

5. Vorrichtung nach Anspruch 1, wobei der Prozessor ferner ausgebildet ist zum:

   Bestimmen einer vorbestimmten Position eines Fingers als eine Lichtausstrahlungsposition basierend auf dem Fingerabdruckmuster; und
   Einschalten der einen oder mehreren Lichtquellen, um die Lichter an der Lichtausstrahlungsposition von der Vielzahl von Lichtquellen der Lichtquellengruppe auszustrahlen.

6. Vorrichtung nach Anspruch 5, wobei der Prozessor ferner zum Bestimmen der Lichtausstrahlungsposition basierend auf einer Anordnung einer Lichtquelle, die den Phasenunterschied maximiert, ausgebildet ist.

7. Vorrichtung nach Anspruch 1, wobei der Prozessor ferner zum Einschalten einer Lichtquelle, die näher an der Kontaktfläche ist als andere Lichtquellen von der Vielzahl von Lichtquellen, ausgebildet ist.

8. Vorrichtung nach Anspruch 1, wobei der Prozessor einen Speicher, ausgebildet zum Erkennen von einzelnen Benutzern basierend auf den erkannten Fingerabdrücken und Speichern des gemessenen Blutdrucks für die einzelnen Benutzer, umfasst.

9. Vorrichtung nach Anspruch 1, wobei der Prozessor eine Pulswellengeschwindigkeit basierend auf dem Phasenunterschied berechnet und den Blutdruck basierend auf einer Beziehung zwischen der berechneten Pulswellengeschwindigkeit und dem Blutdruck schätzt.

**Revendications**

1. Appareil de mesure de la pression artérielle (200) utilisant un processus de sélection de la source lumineuse, l'appareil comprenant :

   un réseau de sources lumineuses (210) comprenant une pluralité de sources lumineuses ;
   un récepteur lumineux configuré pour recevoir les lumières qui ont pénétré à travers l'utilisateur, et acquérir des signaux de photo-pléthysmographie (PPG) à partir des lumières reçues ; et
   un processeur (240) configuré pour allumer sélectivement une ou plusieurs sources lumineuses parmi la pluralité de sources lumineuses pour émettre une ou plusieurs lumières vers un utilisateur, et mesurer une différence de phase entre les signaux PPG acquis, et mesurer une pression sanguine sur la base de la différence de phase mesurée,
   un capteur de reconnaissance d'empreintes digitales configuré pour reconnaître les empreintes digitales,
   dans lequel le processeur est en outre configuré pour allumer sélectivement les une ou plusieurs sources lumineuses en fonction d'au moins une des formes de contact, d'une zone de contact et d'un motif d'empreinte digitale identifié par les

empreintes digitales reconnues.

guine.

2. Appareil selon la revendication 1, dans lequel le processeur est en outre configuré pour sélectionner une première source lumineuse et une deuxième source lumineuse parmi la pluralité de sources lumineuses du réseau de sources lumineuses, dans lequel la deuxième source lumineuse est disposée plus près du récepteur lumineux que la première source lumineuse.

3. Appareil selon la revendication 2, dans lequel le processeur est en outre configuré pour corriger la différence de phase en fonction d'un délai entre les signaux PPG.

4. Appareil selon la revendication 1, dans lequel le processeur est en outre configuré pour fournir à l'utilisateur des informations sur une position de contact appropriée dans laquelle un doigt de l'utilisateur doit être placé pour mesurer la pression artérielle.

5. Appareil selon la revendication 1, dans lequel le processeur est en outre configuré pour :

déterminer une position prédéterminée d'un doigt comme position d'émission de lumière sur la base du motif d'empreinte digitale ; et allumer une ou plusieurs sources lumineuses pour émettre des lumières sur la position d'émission de lumière parmi la pluralité de sources lumineuses du réseau de sources lumineuses.

6. Appareil selon la revendication 5, dans lequel le processeur est en outre configuré pour déterminer la position d'émission de lumière sur la base d'un emplacement d'une source lumineuse qui maximise la différence de phase.

7. Appareil selon la revendication 1, dans lequel le processeur est en outre configuré pour allumer une source lumineuse qui est plus proche de la zone de contact que d'autres sources lumineuses parmi la pluralité de sources lumineuses.

8. Appareil selon la revendication 1, dans lequel le processeur comprend un stockage configuré pour reconnaître les utilisateurs individuels sur la base des empreintes digitales reconnues, et stocker la pression artérielle mesurée pour les utilisateurs individuels.

9. Appareil selon la revendication 1, dans lequel le processeur calcule une vitesse d'onde de pouls sur la base de la différence de phase, et estime la pression sanguine sur la base d'une relation entre la vitesse d'onde de pouls calculée et la pression san-

FIG. 1

LIGHT SOURCE UNIT — 110

PPG SIGNAL ACQUIRER — 120

BLOOD PRESSURE MEASURER — 130

100

FIG. 2

# FIG. 3

FIG. 4

FIG. 5

FIG. 6

SECOND LIGHT SOURCE

LARGE LIGHT DIFFUSION ANGLE

BODY PART

FIRST LIGHT SOURCE

SMALL LIGHT DIFFUSION ANGLE

BODY PART

FIRST LIGHT SOURCE

SECOND LIGHT SOURCE

LIGHT RECEIVER

BODY PART

EP 3 984 450 B1

FIG. 7

Filter Layer (mosaic)

Sensor array

FIG. 8

FIRST PPG

SECOND PPG

PHASE DIFFERENCE

FIG. 9

FIG. 10

BODY PART (e.g., FINGER)

PULSE WAVE
TRANSMISSION

*l*

LIGHT
SOURCE

LIGHT
RECEIVER

PRESSURE SENSOR

# FIG. 11

PRESSURE (mmHg)

120 · 100 · 80 · 60 · 40 · 20 · 0

PULSE WAVE VELOCITY (arb.Unit)/ PRESSURE (mmHg)

360 · 300 · 240 · 180 · 120 · 60 · 0

BELOW ⟵ ⟶ ABOVE

1110

RECOMMENDED RANGE OF TECTILE PRESSURE

Time (sec)

0 · 500 · 1000 · 1500 · 2000 · 1000

1120 · 1130

EP 3 984 450 B1

24

FIG. 12

BODY PART (e.g., FINGER)

PULSE WAVE
TRANSMISSION

TRANSPARENT
TEMPERATURE
SENSOR

FIRST LIGHT
SOURCE

SECOND LIGHT
SOURCE

LIGHT
RECEIVER

# FIG. 13

FIG. 14

FIG. 15

BODY PART (e.g., FINGER)

PULSE WAVE TRANSMISSION

LIGHT RECEIVER

SECOND LIGHT SOURCE

FIRST LIGHT SOURCE

FINGERPRINT RECOGNITION SENSOR

LIGHT SOURCE ARRAY

FIG. 16

FIRST PPG SIGNAL

SECOND PPG SIGNAL

FIRST FILTERED PPG SIGNAL

SECOND FILTERED PPG SIGNAL

FIRST DIFFERENTIAL PPG SIGNAL

SECOND DIFFERENTIAL PPG SIGNAL

Phase delay(PPT) calculation

FIG. 17

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• US 20090043180 A1

**Non-patent literature cited in the description**

• Effects of cuff inflation and deflation on pulse transit time measured from ECG and multi-wavelength PPG. **JING LIU et al.** 2015 37TH ANNUAL INTERNATIONAL CONFERENCE OF THE IEEE ENGINEERING IN MEDICINE AND BIOLOGY SO-CIETY (EMBC). PROCEEDINGS IEEE, 29 August 2015, 5973-5976